(19) Europäisches Patentamt
European Patent Office
Office européen des brevets

(11) Veröffentlichungsnummer : **0 022 975**
**B1**

(12) # EUROPÄISCHE PATENTSCHRIFT

(45) Veröffentlichungstag der Patentschrift : **26.01.83**

(51) Int. Cl.³ : **C 07 D249/08**, A 01 N 43/64

(21) Anmeldenummer : **80103868.8**

(22) Anmeldetag : **08.07.80**

(54) **Triazolyl-alken-Derivate, Verfahren zu ihrer Herstellung und ihre Verwendung als Fungizide.**

(30) Priorität : **21.07.79 DE 2929602**

(43) Veröffentlichungstag der Anmeldung : **28.01.81 Patentblatt 81/04**

(45) Bekanntmachung des Hinweises auf die Patenterteilung : **26.01.83 Patentblatt 83/04**

(84) Benannte Vertragsstaaten : **AT BE CH DE FR GB IT LI NL SE**

(56) Entgegenhaltungen :
**EP A 0 015 387**
**DE A 2 645 617**
**GB A 2 004 276**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Regel, Erik, Ing.grad.**
**Bergerheide 72a**
**D-5600 Wuppertal 1 (DE)**
Erfinder : **Draber, Wilfried, Dr.**
**In den Birken 81**
**D-5600 Wuppertal 1 (DE)**
Erfinder : **Büchel, Karl Heinz, Prof.Dr.**
**Haus an der Dachsdelle Dabringhausener Strasse 42**
**D-5093 Burscheid (DE)**
Erfinder : **Frohberger, Paul-Ernst, Dr.**
**Willi-Baumeisterstrasse 5**
**D-5090 Leverkusen (DE)**
Erfinder : **Brandes, Wilhelm, Dr.**
**Eichendorffstrasse 3**
**D-5623 Leichlingen (DE)**
Erfinder : **Paul, Volker, Dr.**
**Ahornstrasse 5**
**D-5650 Solingen (DE)**

**EP 0 022 975 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Triazolyl-alken-Derivate, Verfahren zu ihrer Herstellung und ihre Verwendung als Fungizide

Die vorliegende Erfindung betrifft neue Triazolyl-alken-Derivate, ein Verfahren zu ihrer Herstellung und ihre Verwendung als Fungizide.

Es ist bereits bekannt geworden, daß Triazolyl-alkan-Derivate, wie beispielsweise 1-(4-Biphenylyloxy)-2-tert.-butylcarbonyloxy-3,3-dimethyl-1-(1,2,4-triazol-1-yl)-butan, gute fungizide Eigenschaften besitzen. (Vergleiche hierzu die DE-OS 26 00 799). Die Wirkung ist jedoch, insbesondere bei niedrigen Aufwandmengen und -konzentrationen, nicht immer ganz befriedigend. Weiterhin sind Triazolyl-phenacyl-styrol-Derivate mit fungizider Wirksamkeit gegen pflanzenschädigende Pilze bekannt geworden (vgl. DE-OS 2 645 617) ; ebenso sind 1-Phenyl-2-azolyl-4,4-dimethyl-1-penten-3-ole beschrieben (vgl. GB-PS 2 004 276), die ebenfalls als fungizide Wirkstoffe von Interesse sind. Die Wirkung dieser Verbindungen gegenüber Fusicladium- und Erysiphe-Arten ist bei niedrigen Aufwandmengen jedoch nicht immer voll zufriedenstellend.

Es wurden neue Triazolyl-alken-Derivate der allgemeinen Formel

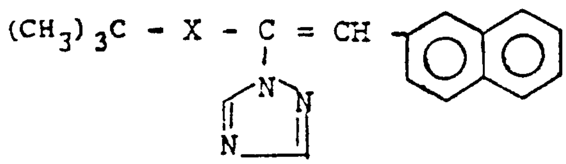

(I)

in welcher

X für die Ketogruppe oder die —CH(OH)—Gruppe steht, sowie deren Säureadditionssalze und Metallsalz-Komplexe gefunden.

Die Verbindungen der Formel (I) können in zwei geometrischen Isomerenformen vorliegen, je nach Anordnung der Gruppen, diean die Doppelbindung gebunden sind. Wenn X für die —CH(OH)—Gruppierung steht, liegt ein asymmetrisches Kohlenstoffatom vor, so daß die Verbindungen der Formel (I) in diesem Fall außerdem in zwei optischen Isomerenformen anfallen.

Die vorliegende Erfindung betrifft sowohl die einzelnen Isomeren als auch Isomeren-Gemische.

Weiterhin wurde gefunden, daß man die Triazolyl-alken-Derivate der Formel (I) sowie deren Säureadditions-Salze und Metallsalz-Komplexe erhält, wenn man ein Triazolylketon der Formel

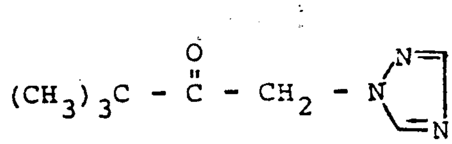

(II)

mit 2-Naphthylaldehyd der Formel

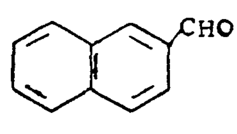

(III)

in Gegenwart eines Lösungsmittels und in Gegenwart eines Katalysators umsetzt ; und

gegebenenfalls die entstehenden Keto-Derivate der Formel (I) in an sich bekannter Weise mit komplexen Hydriden, gegebenenfalls in Gegenwart eines Lösungsmittels, oder mit Aluminium-isopropylat in Gegenwart eines Lösungsmittels reduziert ; und

gegebenenfalls anschließend an die erhaltenen Keto- bzw. reduzierten Derivate der Formel (I) eine Säure oder ein Metallsalz addiert.

Die Triazolyl-alken-Derivate der Formel (I) weisen starke fungizide Eigenschaften auf. Ueberraschenderweise zeigen die erfindungsgemäßen Stoffe eine bessere fungizide Wirksamkeit, als die aus dem Stand der Technik bekannten Triazolyl-alkan-Derivate, wie beispielsweise 1-(4-Biphenylyloxy)-2-tert.-butylcarbonyloxy-3,3-dimethyl-1-(1,2,4-triazol-1-yl)-butan, welches chemisch und wirkungsmäßig naheliegende Verbindungen sind. Die erfindungsgemäßen Stoffe stellen somit eine Bereicherung der Technik dar.

Verwendet man Pinakolyl-1,2,4-triazol und 2-Naphthaldehyd als Ausgangsstoffe, so kann der Reaktionsablauf durch das folgende Formelschema wiedergegeben werden

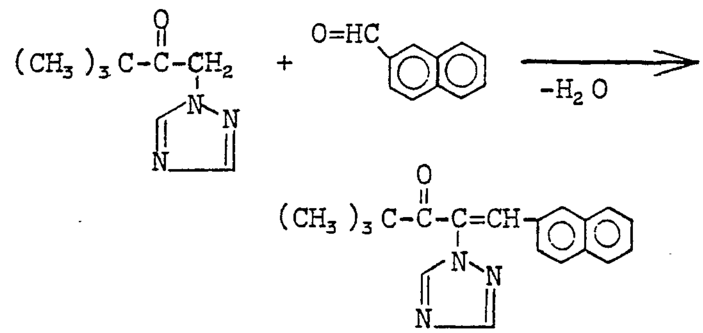


Verwendet man 4,4-Dimethyl-1-(naphth-2-yl)-2-(1,2,4-triazol-1-yl)-1-penten-3-on und Natriumborhydrid als Ausgangsstoffe, so kann der Reaktionsablauf durch das folgende Formelschema wiedergegeben werden

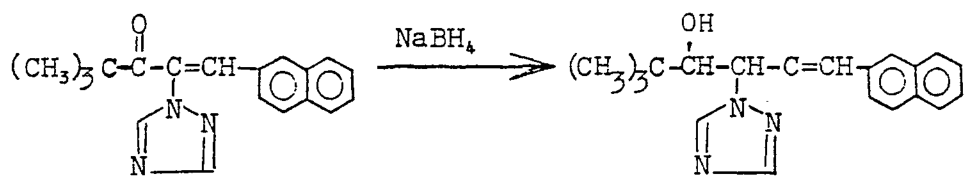


Das bei der Durchführung des erfindungsgemäßen Verfahrens als Ausgangsstoff benötigte Triazolylketon ist durch die Formel (II) definiert. Die Verbindung ist bekannt (vgl. DE-OS 2 431 407, DE-OS 2 610 022 und DE-OS 2 638 470) und kann erhalten werden, indem man das entsprechende Halogen-Keton in Gegenwart eines Säurebinders mit 1,2,4-Triazol umsetzt.

Das außerdem für das erfindungsgemäße Verfahren als Ausgangsstoff zu verwendende 2-Naphthylaldehyd ist durch die Formel (III) definiert. Die Verbindung ist allgemein bekannt.

Die weiterhin gegebenenfalls als Reaktionskomponenten benötigten komplexen Hydride sowie das Aluminiumisopropylat sind allgemein bekannte Verbindungen der organischen Chemie. Als Beispiele für die komplexen Hydride seien vorzugsweise Natriumborhydrid und Lithiumalanat genannt.

Als Lösungsmittel kommen für die erfindungsgemäße Umsetzung vorzugsweise inerte organische Lösungsmittel infrage. Hierzu gehören vorzugsweise Alkohole, wie Methanol und Ethanol ; Ether, wie Tetrahydrofuran und Dioxan ; aliphatische und cycloaliphatische Kohlenwasserstoffe, wie Hexan und Cyclohexan ; aromatische Kohlenwasserstoffe, wie Benzol, Toluol und Xylol ; halogenierte aliphatische und aromatische Kohlenwasserstoffe, wie Methylenchlorid und Tetrachlorkohlenstoff, Chloroform, Chlorbenzol und Dichlorbenzol.

Das erfindungsgemäße Verfahren wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid ; organische Basen, wie Pyridin und Piperidin ; sowie insbesondere Piperidinacetat.

Die Reaktionstemperaturen können bei der Durchführung des erfindungsgemäßen Verfahrens in einem größeren Bereich variiert werden. Im allgemeinen arbeitet man zwischen 20 und 160 °C, vorzugsweise bei der Siedetemperatur des jeweiligen Lösungsmittels.

Bei der Durchführung des erfindungsgemäßen Verfahrens setzt man auf 1 Mol Triazolyl-keton der Formel (II) 1 bis 1,5 Mol 2-Naphthylaldehyd der Formel (III) und katalytische bis 0,2 molare Mengen an Katalysator ein. Die Isolierung der Verbindungen der Formel (I) erfolgt in üblicher Weise.

Wird die erfindungsgemäße Reduktion mit komplexen Hydriden durchgeführt, so kommen als Verdünnungsmittel vorzugsweise polare organische Lösungsmittel infrage. Hierzu gehören vorzugsweise Alkohole, wie Methanol, Ethanol, Butanol, Isopropanol ; sowie Ether, wie Diethylether oder Tetrahydrofuran. Die Reaktion wird im allgemeinen bei 0 bis 30 °C, vorzugsweise bei Raumtemperatur, durchgeführt. Hierzu setzt man auf 1 Mol des Ketons der Formel (I) etwa 1 Mol eines komplexen Hydrids, wie Natriumborhydrid oder Lithiumalanat, ein. Die Isolierung der reduzierten Verbindungen der Formel (I) erfolgt in üblicher Weise.

Arbeitet man mit Aluminiumisopropylat als Reduktionsmittel, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der Formel (I) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage : Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäure, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z.B. die Chlorwasserstoffsäure und die Bromwasserstoffsäure, ferner Phosphorsäure, Salpetersäure und Schwefelsäure.

Die Metall-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Stoffe mit besonders gutem Erfolg zur Bekämpfung von Getreidekrankheiten, wie Getreidemehltau und Gerstenmehltau, eingesetzt werden.

In bestimmten Aufwandmengen zeigen die erfindungsgemäßen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bakannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen. Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

In dem nachfolgenden Beispiel wird die nachstehend angegebene Verbindungen als Vergleichssubstanz eingesetzt

(A) 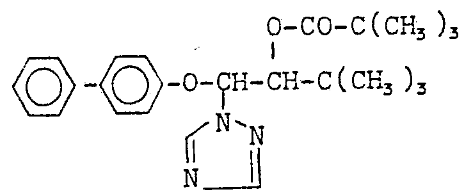

Beispiel A

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkylaryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 bis 22 °C und einer Luftfeuchtigkeit von 80 bis 90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

In diesem Test zeigen z.B. die folgenden Verbindungen eine sehr gute Wirkung, die derjenigen der aus dem Stand der Technik bekannten Verbindung (A) überlegen ist:

Verbindung gemäß Herstellungsbeispiel 2.

Herstellungsbeispiele

Beispiel 1

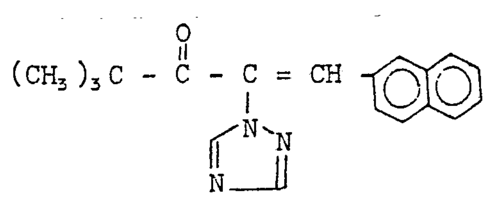

16,7 g (0,1 Mol) Pinakolyl-1,2,4-triazol, 15,6 g (0,1 Mol) 2-Naphthaldehyd, 1 g Piperidin und 3 g Eisessig in 50 ml Toluol werden am Wasserabscheider unter Rückfluß erhitzt, bis kein Wasser mehr übergeht. Nach dem Abkühlen der Reaktionslösung wird diese durch Zugabe von Ether verdünnt und mit Wasser gewaschen. Die organische Phase wird über Natriumsulfat getrocknet und durch Abdestillieren der Lösungsmittel im Vakuum eingeengt. Der Rückstand wird im Vakuum destilliert. Man erhält 18 g (59 % der Theorie) 4,4-Dimethyl-1-(naphth-2-yl)-2-(1,2,4-triazol-1-yl)-1-penten-3-on vom Siedepunkt 220 °C.

Herstellung des Ausgangsproduktes

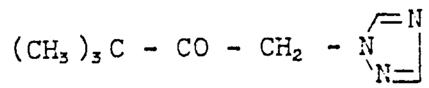

138 g (2 Mol) 1,2,4-Triazol werden bei Raumtemperatur portionsweise zu 276,4 g (2 Mol) gemahlenem Kaliumcarbonat und 296,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72 % der Theorie) 3,3-Dimethyl-1-(1,2,4-triazol-1-yl)-butan-2-on vom Schmelzpunkt 62-64 °C.

Beispiel 2

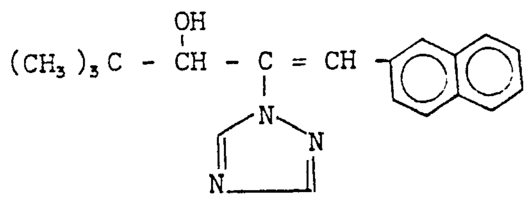

85 g (0,27 Mol) 4,4-Dimethyl-1-(naphth-2-yl)-2-(1,2,4-triazol-1-yl)-1-penten-3-on (Beispiel 1) werden in 200 ml Methanol aufgenommen und unter Rühren und Kühlen portionsweise mit 6 g Natriumborhydrid versetzt. Nach beendeter Reaktion wird das Reaktionsgemisch auf pH-6 eingestellt und eingeengt. Der Rückstand wird in Methylenchlorid aufgenommen, mit gesättigter Natriumhydrogencarbonat-Lösung gewaschen, über Natriumsulfat getrocknet, filtriert und eingeengt. Der ölige Rückstand wird durch Zusatz von Ether zur Kristallisation gebracht. Man erhält 41 g (49 % der Theorie) 4,4-Dimethyl-1-(naphth-2-yl)-2-(1,2,4-triazol-1-yl)-1-penten-3-ol vom Schmelzpunkt 122-124 °C.

**Ansprüche**

1. Triazolyl-alken-Derivate der allgemeinen Formel

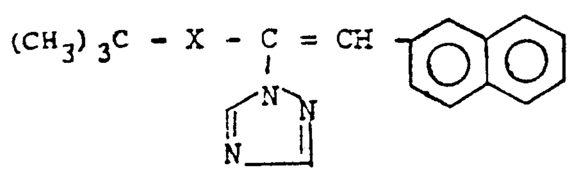

(I)

in welcher

X für die Ketogruppe oder die —CH(OH)—Gruppe steht, sowie deren Säureadditionssalze und Metallsalz-Komplexe.

2. Verfahren zur Herstellung von Triazolyl-alken-Derivaten der allgemeinen Formel

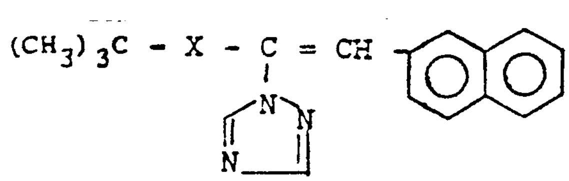

(I)

in welcher

X für die Ketogruppe oder die —CH(OH)—Gruppe steht, sowie von deren Säureadditionssalzen und Metallsalz-Komplexen, dadurch gekennzeichnet, daß man ein Triazolyl-keton der Formel

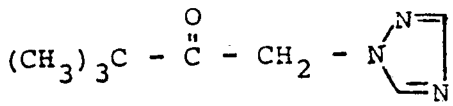

(II)

mit 2-Naphthylaldehyd der Formel

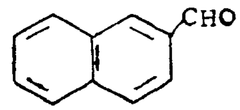

(III)

in Gegenwart eines Lösungsmittels und in Gegenwart eines Katalysators umsetzt ; und gegebenenfalls die entstehenden Keto-Derivate der Formel (I) in an sich bekannter Weise mit komplexen Hydriden, gegebenenfalls in Gegenwart eines Lösungsmittels, oder mit Aluminium-isopropylat in Gegenwart eines Lösungsmittels reduziert ; und gegebenenfalls anschließend an die erhaltenen Keto- bzw. reduzierten Derivate der Formel (I) eine Säure oder ein Metallsalz addiert.

3. Fungizide Mittel, gekennzeichnet durch einen Gehalt an mindestens einem Triazolyl-alken-Derivat der Formel (I).

4. Verwendung von Triazolyl-alken-Derivaten der Formel (I) zur Bekämpfung von Pilzen.

**Claims**

1. Triazolyl-alkene derivatives of the general formula

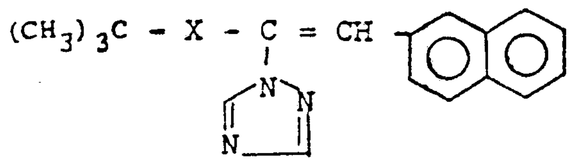

(I)

in which

X represents the keto group or the —CH(OH)— group, and acid addition salts and metal salt complexes thereof.

2. Process for the preparation of triazolyl-alkene derivatives of the general formula

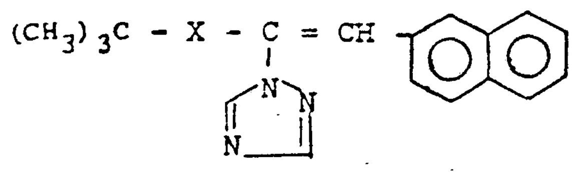

(I)

in which

X represents the keto group or the —CH(OH)— group, and of acid addition salts and metal salt complexes thereof, characterised in that a triazolyl ketone of the formula

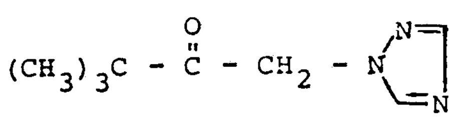

(II)

is reacted with 2-naphthylaldehyde of the formula

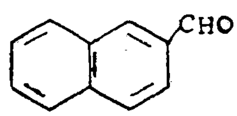

(III)

in the presence of a solvent and in the presence of a catalyst ; and the keto derivatives formed, of the formula (I), are optionally reduced in a manner which is in itself known with complex hydrides, if appropriate in the presence of a solvent, or with aluminium isopropylate, in the presence of a solvent ; and an acid or a metal salt is then optionally added onto the resulting keto derivatives or reduced derivatives of the formula (I).

3. Fungicidal agents, characterised in that they contain at least one triazolyl-alkene derivative of the formula (I).

4. Use of triazolyl-alkene derivatives of the formula (I) for combating fungi.

**Revendications**

1. Dérivés de triazolyl-alcènes de formule générale

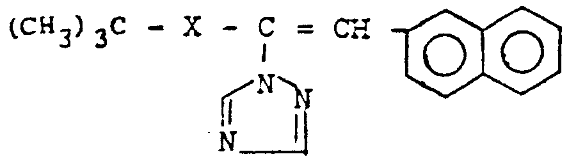

(I)

dans laquelle

X représente le groupe céto ou le groupe —CH(OH)—, et leurs sels formés par addition avec des acides et complexes de sels métalliques.

2. Procédé de préparation des dérivés de triazolyl-alcènes de formule générale

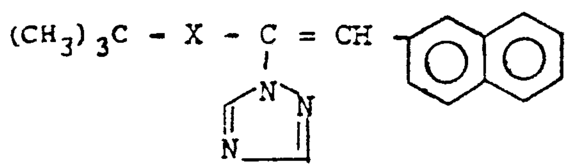

(I)

dans laquelle

X représente le groupe céto ou le groupe —CH(OH)—, et de leurs sels formés par addition avec des acides et complexes de sels métalliques, caractérisé en ce que l'on fait réagir une triazolyl-cétone de formule

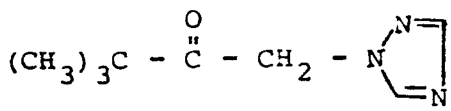

(II)

avec le 2-naphtaldéhyde de formule

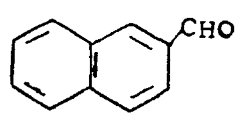

(III)

en présence d'un solvant et en présence d'un catalyseur ; et le cas échéant, on réduit les dérivés cétoniques formés de formule I, de manière connue en soi, à l'aide d'hydrures complexes, éventuellement en présence d'un solvant ou à l'aide de l'isopropylate d'aluminium en présence d'un solvant ; et le cas échéant, on fixe ensuite par addition un acide ou un sel métallique sur les dérivés cétoniques ou réduits de formule I ainsi obtenus.

3. Produits fongicides caractérisés en ce qu'ils contiennent au moins un dérivé de triazolyl-alcène de formule I.

4. Utilisation des dérivés de triazolyl-alcènes de formule I dans la lutte contre les mycètes.